(19) Europäisches Patentamt
European Patent Office
Office européen des brevets

(11) EP 0 877 596 B1

(12) EUROPEAN PATENT SPECIFICATION

(45) Date of publication and mention of the grant of the patent:
**04.07.2001 Bulletin 2001/27**

(21) Application number: **97904881.6**

(22) Date of filing: **27.01.1997**

(51) Int Cl.$^7$: **A61K 7/50**, A61K 7/16, A61K 7/06, A61K 7/48

(86) International application number:
**PCT/US97/01272**

(87) International publication number:
**WO 97/27839 (07.08.1997 Gazette 1997/34)**

# (54) CLEANSING COMPOSITION COMPRISING COLOR STABILIZER(S) AND SURFACTANT(S)

REINIGUNGSMITTEL, ENTHALTEND FARBSTABILISATOR(EN) UND TENSID(E)

COMPOSITION DE NETTOYAGE COMPRENANT UN OU PLUSIEURS STABILISANTS DES COULEURS ET UN OU PLUSIEURS TENSIOACTIFS

(84) Designated Contracting States:
**AT BE CH DE DK ES FI FR GB GR IE IT LI NL PT SE**
Designated Extension States:
**RO**

(30) Priority: **30.01.1996 US 10796 P**
**30.01.1996 US 10840 P**
**30.01.1996 US 10841 P**

(43) Date of publication of application:
**18.11.1998 Bulletin 1998/47**

(73) Proprietor: **Colgate-Palmolive Company**
**New York, N.Y. 10022 (US)**

(72) Inventors:
- **PAYNE, Richard, K.**
  **Brielle, NJ 08736 (US)**
- **HWANG, AnBen**
  **Verona, NJ 07044 (US)**
- **SUBRAMANYAN, Ravi**
  **Belle Mead, NJ 08502 (US)**

(74) Representative:
**Smulders, Theodorus A.H.J., Ir. et al**
**Vereenigde**
**Postbus 87930**
**2508 DH Den Haag (NL)**

(56) References cited:
**WO-A-93/23515**
**DE-A- 4 412 235**
**DE-A- 19 516 698**
**US-A- 4 077 911**
**US-A- 4 282 163**

- **DATABASE WPI Week 9608 Derwent Publications Ltd., London, GB; AN 96-074827 XP002031488 "Stabilising method of monoethanolamine - comprises adding antioxidant to monoethanolamine or solvent contg. monoethanolamine and gives high deterioration resistance to cleaning agents" & JP 07 330 692 A (MITSUI TOATSU ) , 19 December 1995**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 877 596 B1

Printed by Jouve, 75001 PARIS (FR)

## Description

### BACKGROUND OF THE INVENTION

Soap has been used for time memorial to cleanse human skin. Both solid and liquid compositions containing soap have been used to deliver the surfactant to the skin for cleansing purposes. As with virtually every composition used for consumer purposes, the stability of the composition is critical for proper use and/or prolonged storage prior to use.

It is well known that free radical reactions will interfere with the stability of soap containing compositions. Free radicals initiate chain reactions which bring about the deterioration of long chain hydrocarbon materials such as soaps, free fatty acids, synthetic surfactants and the like present in cleansing compositions. Such reactions can bring about among other observable effects, color changes in the cleansing composition and eventually the rancidification of the formulation caused by the presence of breakdown products which are highly odiferous.

The deterioration of the long chain hydrocarbon containing materials can be substantially hindered by the use of known materials to either hinder the catalyzation of certain free radical mechanisms or work as a free radical sink to terminate the free radical chain reaction by rendering the chain free radical harmless. Example of the former type of materials are agents which chelate metals such as copper and iron which are known catalyzers of free radical initiation steps, particularly at points of unsaturation in the hydrocarbon chain. Such agents include ethylene diamine tetra carboxylic acid and its salts and various salts of phosphonic acid derivatives. Exemplary of the free radical sinks are various aromatic compounds which seems to work as a sink for the generated free radials whatever their initial mode of propagation might be. A prime example of this type of compound which has been in use for several decades is butylated hydroxy toluene, (usually known by its abbreviation BHT).

However, even with the use of BHT, stability problems are known to occur. General discoloration of cleansing bars occurs from time-to-time as well as specific points of coloring, usually intensely yellow also occurs. Although unusual, after prolonged periods of time, odors from a soap bar can also occur. Therefore, a need for a better cleansing formulation, particularly solid, stability enhancing additive remains.

### SUMMARY OF THE INVENTION

In accordance with the invention, there is a cleansing composition which comprises a cleansing effective amount of a long chain alkyl or alkenyl containing surfactant, wherein by long chain is meant at least 8 carbon atoms, or mixtures thereof and a color stabilizing effective amount of a compound of the formula selected from the group consisting of

a.

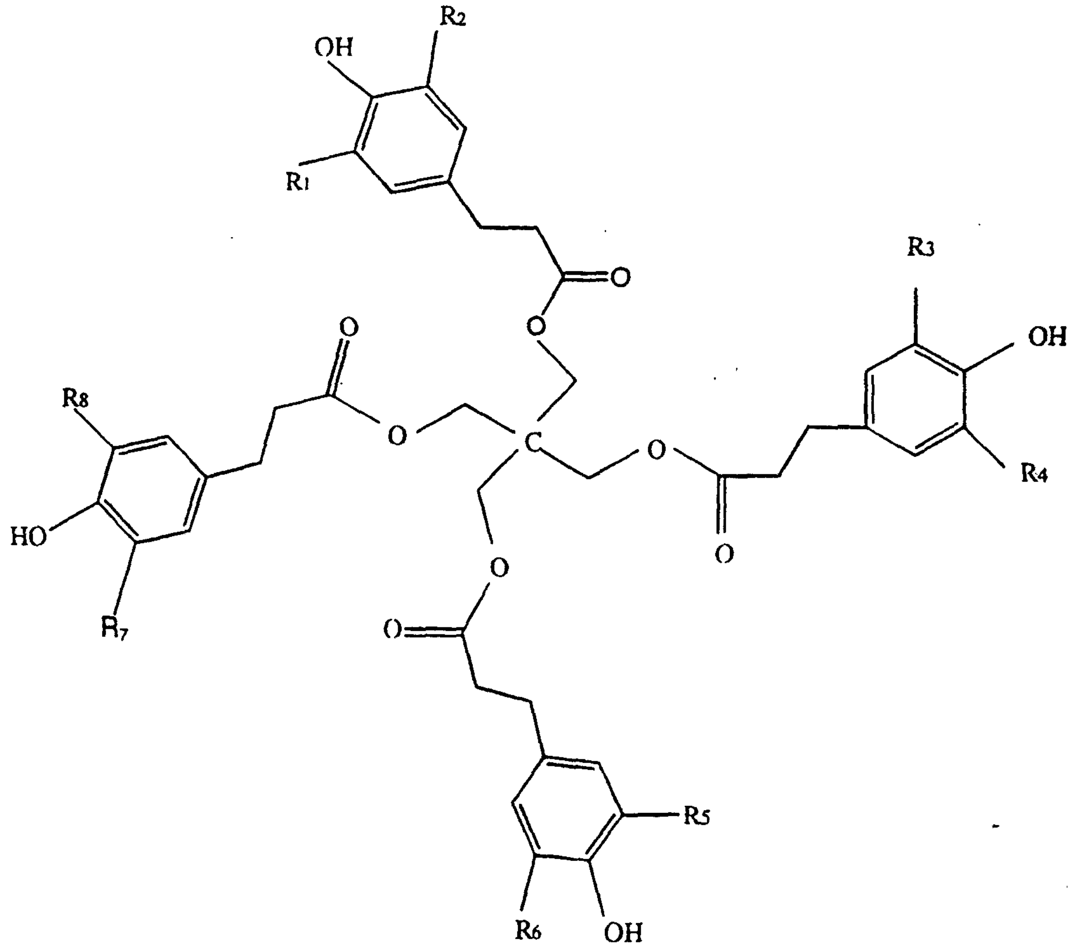

wherein $R_1$, $R_2$, $R_3$, $R_4$, $R_5$, $R_6$, $R_7$ and $R_8$ are the same or different and are isopropyl or tertiary butyl,

b.

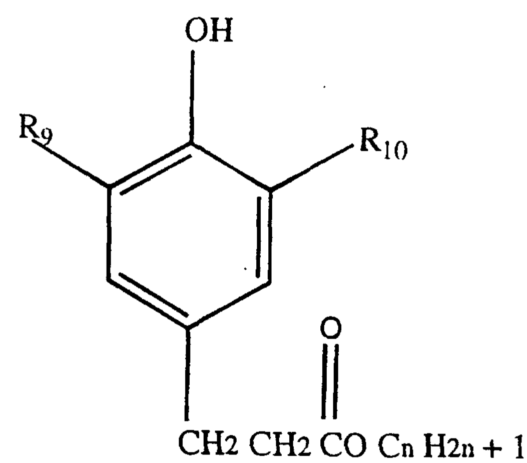

wherein $R_9$ and $R_{10}$ are the same or different and are isopropyl or tertiary butyl and n is 8 to 20, or

c.

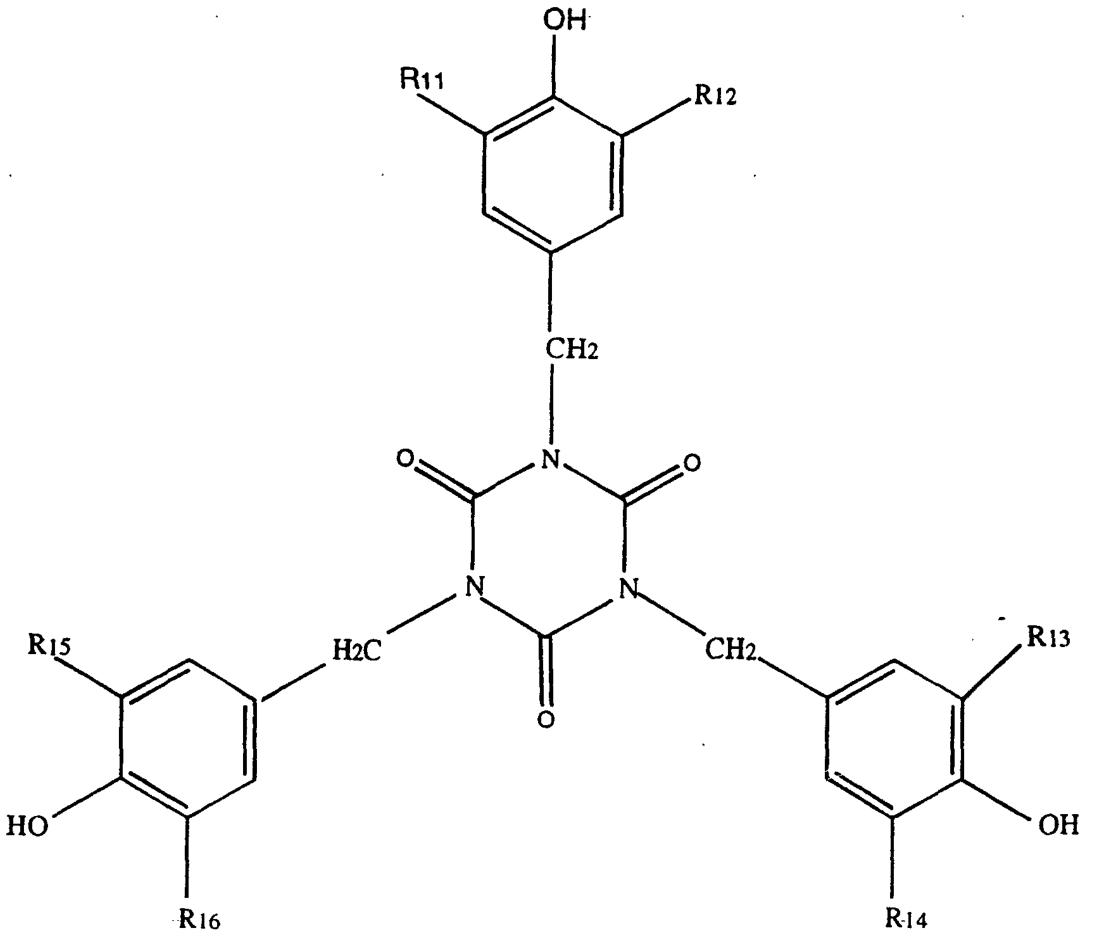

wherein $R_{11}$, $R_{12}$, $R_{13}$, $R_{14}$, $R_{15}$ and $R_{16}$ are the same or different and are isopropyl or tertiary butyl.

A preferred composition is one wherein the surfactant is a soap.

A further preferred composition is a solid, more preferably a bar.

DETAILED DESCRIPTION OF THE INVENTION

Any surfactant may be used in the cleansing composition which removes soil from skin and which is susceptible to degradation upon prolonged shelf-life, particularly with respect to degradation which leads to discoloration and/or unpleasant odors. Therefore, any surfactant which has a long chain alkyl or alkenyl group or mixtures thereof, can be susceptible to such degradation. By long chain is meant at least about 8 carbon atoms, preferably 10, usually normal or having a slight amount of branching. Generally, the maximum number of carbon atoms is not significant but usually above 20 is not preferred. Small quantities of olefinic bond(s) can be present in the predominantly alkyl sections, particularly if the source of the "alkyl" group is obtained from a natural product such as tallow, coconut oil and the like.

Soap, a long chain alkyl or alkenyl, branched or normal carboxylic acid salt such as sodium, potassium, ammonium or substituted ammonium salt can be present in the composition and is preferred.

Examples of such surfactants are the anionic, amphoteric, nonionic and cationic surfactants. Examples of anionic zwitterionic surfactants include but are not limited to alkyl sulfates, anionic acyl sarcosinates, methyl acyl taurates, N-acyl glutamates, acyl isethionates, alkyl sulfosuccinates, alkyl phosphate esters, ethoxylated alkyl phosphate esters, trideceth sulfates, protein condensates, mixtures of ethoxylated alkyl sulfates and the like.

Anionic nonsoap surfactants can be exemplified by the alkali metal salts of organic sulfate having in their molecular structure an alkyl radical containing from about 8 to about 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$-$C_{18}$ carbon atoms), sodium coconut oil fatty acid monoglyceride sulfates and sulfonates; sodium or potassium salts of

sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art.

Zwitterionic surfactants can be exemplified by those which can be broadly described as derivatives of aliphatic quaternary ammonium, phosphonium, and sulfonium compounds, in which the aliphatic radicals can be straight chain or branched and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

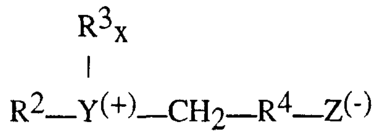

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to about 3 carbon atoms; X is 1 when Y is a sulfur atom and 2 when Y is a nitrogen or phosphorus atom, $R^4$ is an alkylene or hydroxyalkylene of from 0 to about 4 carbon atoms and Z is a radical selected from the group consisting of carboxylate, sulfonate, sulfate, phosphonate, and phosphate groups.

Examples include: 4-[N,N-di(2-hydroxyethyl)-N-octadecylammonio]-butane-1-carboxylate; 5-[S-3-hydroxypropyl-S-hexadecylsulfonio] -3 hydroxypentane-1-sulfate; 3-[P,P-P-diethyl-P 3,6,9 trioxatetradecyl- phosphonio]-2-hydroxypropane-1-phosphate; 3-[N,N-dipropyl-N-3 dodecoxy-2-hydroxypropylammonio]-propane-1-phosphonate; 3-(N,N-di- methyl-N-hexadecylammonio) propane-1-sulfonate; 3-(N,N-dimethyl-N-hexadecylammonio)-2-hydroxypropane-1-sulfonate; 4-(N,N-di(2-hydroxyethyl)-N-(2 hydroxydodecyl) ammonio]-butane-1-carboxylate; 3-[S-ethyl-S-(3-dodecoxy-2-hydroxypropyl)sulfonio]-propane-1-phosphate; 3-(P,P-dimethyl-P-dodecylphosphonio)-propane-1-phosphonate; and 5-[N,N-di(3-hydroxypropyl)-N-hexadecylammonio]-2-hydroxy-pentane- 1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxylate, sulfonate, sulfate, phosphate, or phosphonate. Examples of compounds falling within this definition are sodium 3-dodecylaminopropionate, sodium 3-dodecylaminopropane sulfonate, N-alkyltaurines, such as the one prepared by reacting dodecylamine with sodium isethionate according to the teaching of U.S. Patent No.2,658,072, N-higher alkyl aspartic acids, such as those produced according to the teaching of U.S. Patent No. 2,438,091, and the products sold under the trade name "Miranol" and described in U.S. Patent No. 2,528,378. Other amphoterics such as betaines are also useful in the present composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl) carboxymethyl betaine, oleyl dimethyl gamma-carboxypropyl betaine, lauryl bis-(2-hydro-xypropyl) alpha-carboxyethyl betaine, etc. The sulfobetaines may be represented by coco dimethyl sulfopropyl betaine, stearyl dimethyl sulfopropyl betaine, amido betaines, amidosulfobetaines, and the like.

Many cationic surfactants are known to the art. By way of example, the following may be mentioned:

- stearyldimenthylbenzyl ammonium chloride;
- dodecyltrimethylammonium chloride;
- nonylbenzylethyldimethyl ammonium nitrate;
- tetradecylpyridinium bromide;
- laurylpyridinium chloride;
- cetylpyridinium chloride
- laurylpyridinium chloride;

- laurylisoquinolium bromide;
- ditallow(Hydrogenated)dimethyl ammonium chloride;
- dilauryldimethyl ammonium chloride; and
- stearalkonium chloride.

Additional cationic surfactants are disclosed in USP 4,303,543 see column 4, lines 58 and column 5, lines 1-42, incorporated herein by references. Also see CTFA Cosmetic Ingredient Dictionary, 4th Edition 1991, pages 509-514 for various long chain alkyl cationic surfactants: incorporated herein by references.

Nonionic surfactants can be broadly defined as compounds produced by the condensation of alkylene oxide groups (hydrophilic in nature) with an organic hydrophobic compound. which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.
2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.
3. The condensation product of aliphatic alcohols having from 8 to 18 carbon atoms, in either straight chain or branched chain configuration with ethylene oxide, e.g., a coconut alcohol ethylene oxide condensate having from 10 to 30 moles of ethylene oxide per mole of coconut alcohol, the coconut alcohol fraction having from 10 to 14 carbon atoms. Other ethylene oxide condensation products are ethoxylated fatty acid esters of polyhydric alcohols (e.g., Tween 20-polyoxyethylene (20) sorbitan monolaurate).
4. Long chain tertiary amine oxides corresponding to the following general formula:

$$R_1R_2R_3N \rightarrow 0$$

wherein $R_1$ contains an alkyl, alkenyl or monohydroxy alkyl radical of from 8 to 18 carbon atoms, from 0 to 10 ethylene oxide moieties, and from 0 to 1 glyceryl moiety, and, $R_2$ and $R_3$ contain from 1 to 3 carbon atoms and from 0 to 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyl-di(2-hydroxyethyl) amine oxide, dimethyloctylamine oxide, dimethyldecylamine oxide, dimethyltetradecylamine oxide, 3,6,9 trioxaheptadecyldiethylamine oxide. di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.
5. Long chain tertiary phosphine oxides corresponding to the following general formula:

$$RR'R''P \rightarrow 0$$

wherein R contains an alkyl, alkenyl or monohydroxyalkyl radical ranging from 8 to 20 carbon atoms in chain length, from 0 to 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecylmethylethylphosphine oxide, 3,6,9-trioxaoctadecyldimethylphosphine oxide, cetyldimethylphosphine oxide, 3-dodecoxy-2-hydroxypropyldi(2-hydroxyethyl) phosphine oxide stearyldimethylphosphine oxide, cetylethyl propylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl) phosphine oxide, tetradecylmethyl-2-hydroxypropylphosphine oxide, oleyldimethylphosphine oxide, 2-hydroxydodecyldimethylphosphine oxide.
6. Long chain dialkyl sulfoxides containing one short chain alkyl or hydroxy alkyl radical of 1 to 3 carbon atoms (usually methyl) and one long hydrophobic chain which contain alkyl, alkenyl, hydroxy alkyl, or keto alkyl radicals

containing from 8 to 20 carbon atoms, from 0 to 10 ethylene oxide moieties and from 0 to 1 glyceryl moiety. Examples include: octadecyl methyl sulfoxide, 2-ketotridecyl methyl sulfoxide, 3,6,9-trioxaoctadecyl 2-hydroxyethyl sulfoxide, dodecyl methyl sulfoxide. oleyl 3-hydroxypropyl sulfoxide, tetradecyl methyl sulfoxide, 3 methoxytridecylmethyl sulfoxide, 3-hydroxytridecyl methyl sulfoxide, 3-hydroxy-4-dodecoxybutyl methyl sulfoxide.

Any quantity of surfactant or mixture of surfactant which brings about a skin cleansing effect can be employed in the composition of this invention. Generally, at least about 1 wt.% of the composition should be surfactant. Preferred minimums of at least 3, 5, 7, 10, 20 and 30 wt.% surfactant(s) can be present in the composition. Maximum quantities of surfactant(s) depends upon the physical nature of the composition being employed. Generally, no more than 95-97 wt.% surfactant(s) are present, specifically no more than about 90 wt.% surfactant(s). Maximum quantities of 20, 30, 40, 50, 60, 70, 80, or 85 wt.% surfactant(s) can also be readily employed.

The anionic surfactant can be present in the composition in various preferred quantities beyond those general quantities previously discussed for all surfactants of from 1 to 96 wt.%, specifically 5 to 85 wt.%. With respect to liquid, preferably aqueous, compositions, the anionic surfactant(s) is from 2 to 20 wt.% of the composition, specifically 5 to 15 wt.%. For a solid composition, the anionic surfactant(s) can be from 5 to 90 wt.%, preferably from 10 to 50 wt.% for a "syndet" bar, 55 to 80 wt.% for a "combar", and 70 to 90 wt.%, more preferably 75 to 85 wt.% in a solid composition wherein there is only one anionic surfactant therein, such as soap.

The antioxidant of the formula is specifically one where all the "R" groups are the same or different and preferably the same in each group a, b or c and most preferably, are all tertiary butyl. When all the "R" groups are tertiary butyl, the compound in group a is available from Ciba-Geigy as Irganox 1010. The CAS number is 6683-19-8 and its chemical name is tetrakis [Methylene (3,5-di-tert-butyl-4-hydroxyhydrocinnamate)] methane. In group b, when the "R" groups are each tertiary butyl and n is 18, the compound is available from Ciba-Geigy as Irganox 1076. The CAS number is 2082-79-3 and its chemical name is octadecyl (3,5-di-tert-butyl-4-hydroxyhydrocinnamate). In group c, when all the "R" groups are tertiary butyl, the compound is available from Ciba-Geigy as Irganox 3114. The CAS number is 2767-62-6 and its chemical name is tris-(3,5-di-tert. butyl-4-hydroxybenzyl) isocyanurate. The quantity of antioxidant percent in the compositions(s) of the invention is an amount effective to provide color stability to the composition upon shelf aging. The upper limit appears to be primarily dependent upon the cost of the material. In solid compositions, generally from 25 to 500 ppm of the composition can be employed, preferably from 50 to 300 ppm of the composition. In liquid compositions generally from 10 to 300 ppm of the antioxidant can be employed, preferably from 25 to 250 ppm of the composition.

The physical nature of the composition is not critical and can be a solid, liquid or gel. The method of making such a composition is by the usual methods employed in the detergent industry. The antioxidant is added at the usual point an antioxidant is added in the process.

Below are standard liquid and solid compositions as illustrative examples of the composition(s).

| Solid Soap Bar Formula | |
|---|---|
| **Component** | **% By Weight** |
| **Mixture of Sodium Tallowate, Cocoate, Palmate, and Palm Kernelate soaps** | **80 - 87** |
| **Water** | **8 - 10** |
| **Glycerine** | **0.5 - 1.5** |
| **Fragrance/Dyes** | **Q.S.** |
| **Versenex 80-DTPA (40%)** | **0.2** |
| **Titanium Dioxide** | **0.2** |
| **Citric Acid (50%)** | **0.25** |
| **Sodium Chloride** | **0.05 - 1.3** |
| **Antioxidant (Irganox 1010 or Irganox 1076 or Irganox 3114)** | **0.0025-0.1** |

| Liquid | |
|---|---|
| **Component** | **% By Weight** |
| Alpha Olefin Sulfonate (40%) | 10.0 |
| Ammonium Lauryl Sulfate (28%) | 25.0 |
| Cocamidopropyl Hydroxy Sultaine (50%) | 3.5 |
| Versene 100 (Dow) EDTA (39%) | 0.2 |
| Propylene Glycol | 1.0 |
| Lauryl diethanolamide | 0.5 |
| Chloroxylenol | 1.0 |
| Citric Acid | Q.S. to pH 5.5-6.5 |
| Fragrance, Dye(s), Preservative | Q.S. |
| Sodium Chloride | 0.05-0.50 |
| Water | 58.5 |
| Antioxidant (Irganox 1010 or Irganox 1076 or Irganox 3114) | 0.001-0.05 |

The compounds of the invention are not restricted to only personal care cleansing compositions. These compounds can also be useful in oral compositions designed for cleansing teeth.

Organic surface-active agents are used in oral compositions to achieve increased prophylactic action, assist in achieving thorough and complete dispersion of any active material such as an anticalculus agent throughout the oral cavity, as well as rendering the compositions more cosmetically acceptable. The organic surface-active material is preferably anionic, nonionic, or ampholytic in nature, and it is preferred to employ as the surface-active agent a detersive material which imparts to the composition detersive and foaming properties. Suitable examples of anionic surfactants are water-soluble salts or higher fatty acid monoglyceride monosulfates, such as the sodium salt of the monosulfated monoglyceride of hydrogenated coconut oil fatty acids, higher alkyl sulfates such as sodium lauryl sulfate, alkyl aryl sulfonates such as sodium dodecyl benzene sulfonate, higher alkyl sulfoacetates, higher fatty acid esters of 1,2 dihydroxy propane sulfonates, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium. and ethanolamine salts of N-lauroyl, N-myristoyl, or N-palmittoyl sarcosine which should he substantially free from soap or similar higher fatty acid material. The use of such sarcosinate compounds in certain oral compositions is particularly advantageous since these materials exhibit a prolonged and marked effect in the inhibition of acid formation in the oral cavity due to carbohydrate break down in addition to exerting some reduction in the solubility of tooth enamel in acid solutions.

Examples of water-soluble nonionic surfactants are condensation products of ethylene oxide with various reactive hydrogen-containing compounds reactive therewith having long hydrophobic chains (e.g. aliphatic chains of 12 to 20 carbon atoms), which condensation products ("ethoxamers") contain hydrophilic polyoxyethylene moieties, such as condensation products of poly (ethylene oxide) with fatty acids, fatty alcohols, fatty amides, polyhydric alcohols (e.g. sorbitan monostearate) and polypropyleneoixde (e.g. Pluronic materials).

Generally, surfactants are present in the paste, gel or the typical teeth cleansing composition in from 1 to 15 wt% of the composition. The antioxidant of the present invention can also provide stabilization properties to oral compositions containing a peroxide such as hydrogen peroxide or calcium peroxide and the like. The quantity of antioxidant employed is that amount sufficient to stabilize the composition, for example, as a free radical scavenger. Exemplary of such quantities are antioxidant of from 50 to 5000 ppm, preferably 100 to 2000 ppm of the composition.

The following arc examples of the invention which are intended to illustrate the extent of the invention and not be unduly limited thereof.

The following test system was initially employed to evaluate materials which might be stable in an oxidative environment and suitable for preserving cleansing products, particularly soap containing systems.

**TEST METHOD:**

An equal amount of potential antioxidant Irganox 1010 under evaluation and benzoyl peroxide (weight basis) are dissolved into an inert organic solvent such as chloroform to form a 1% (each) solution. 10 ml. of the solution is

transferred into a round bottom flask, and heated to hoiling under a water cooled condenser. The content is kept refluxing for 15 hours. After removal from heat and allowing to cool to room temperature, the solution is visually inspected for discoloration. This system is designed to bring about yellowing and discoloration which is normally observed in BHT containing cleansing systems.

**TEST RESULT:**

The following materials evaluated show a varied degree of color instability:

Irganox 1010, Irganox 1076 and Irganox 3114 are all more stable than Irganox 1330, which is more stable than Casamine OTB which is more stable than BHT.

a. Irganox 1330 is 1,3,5-trimethyl-2,4,6-tris(3,5-di-tert. butyl-4-hydroxybenzyl) benzene.
b. Casamine OTB is ortho-tolyl biguanide.

The BHT is bright yellow after the test period. The Irganox 1330 and Casamine OTB have varying coloration. Neither Irganox 1010 nor Irganox 1076 show any discoloration after the test period. Irganox 3114 does not show any significant discoloration after the test period.

A methodology as outlined below was utilized for assessing the color stability of antioxidants in soap formulations.

**TEST METHOD:**

250 ppm each of the antioxidant, Irganox 1010 and benzoyl peroxide and 0.2% $TiO_2$ are homogeneously milled into a soap base. The homogeneous mix is extruded through a plodder to produce a billet which is then pressed in a box shaped die to yield 90 mg. bars. The test bars are rapidly aged in an oven maintained at constant 43 degree C for 4 weeks, and one bar is retrieved every week to evaluate for color change. The change in color of the soap bars is measured using a reflectance type colorimeter which employs the C.I.E. LAB color space scale ($L^*a^*b^*$). Where $L^*$ indicates the degree of lightness (white, black), $a^*$ is the Color Space coordinate defining the red/green and $b^*$ is the Color Space coordinate defining the yellow/blue. Freshly prepared bars are pre-read to obtain the initial or standard value. Every seven days bars are read to determine the change in lightness and color. The $DL^*$, $Da^*$ and $Db^*$ values are the difference in color between the initial and aged samples. The $DE^*$ value is a number, calculated from the Color Difference Equation, representing the total color difference (i.e., incorporates lightness and color). Both the $Db^*$ and $DE^*$ values are monitored to study the color stability of a particular antioxidant in the soap bars. A positive $Db^*$ value is indicative of an increase in yellowing, or poor antioxidant color stability. A positive $DE^*$ value suggests an increase in the darkness and color of the soap. A more positive value for this measurement indicates a decrease in oxidative stability of the antioxidant.

The Color Difference Equation ($DE^*$) is as follows:

$$DE^* = (DL^{*2} + Db^{*2} + Da^{*2})^{1/2}$$

**RESULTS:**

Irganox 1010 versus the control bar containing BHT show the following trend:

DE* value: Irganox 1010 <<< BHT
Db* value: Irganox 1010 <<< BHT

Irganox 1010 exhibits good color stability when exposed to strong oxidative environment.

**TEST METHOD:**

Irganox 1010 stability under hydrolytic condition is studied. When boiling as an ethanolic solution in the presence of a strong inorganic base such as sodium hydroxide, Irganox 1010 can be easily hydrolyzed into the corresponding alcohol and carboxylic acid.

Three soap base formulations are prepared to evaluate the hydrolytic stability of Irganox 1010.

(A) Control - Soap chip from typical kettle soap, with 10-13% moisture and 0.02-0.10% free alkalinity as $Na_2O$.

(B) Experiment 1 - Addition of 0.25% citric acid to the above soap chips and thoroughly mixing by passing through a laboratory mill.

(C) Experiment 2 - Addition of 7% fatty acids blend such as a 50:50 wt. mixture of stearic acid and hydrogenated coco fatty acids to the above soap chips and thoroughly mixing by passing through a laboratory mill.

Irganox 1010 is then intimately blended into each lot of soap at 200 ppm. level. The blends were passed through a laboratory mill repeatedly to insure homogeneity and made into thin ribbons. Each batch of soap ribbon is sealed in three separate glass jars and stored in an oven maintained at 43 degree C for 4 weeks. Each week, a 2 gram sample of each soap batch is retrieved from the glass jars and analyzed for concentration of Irganox 1010 by an HPLC procedure.

| **RESULTS:** (Expressed in ppm Irganox 1010) | | | | |
|---|---|---|---|---|
| | 1 Week | 2 Weeks | 3 Weeks | 4 Weeks |
| Control | **211** | **170** | **130** | **95** |
| Experiment 1 | **212** | **198** | **198** | **182** |
| Experiment 2 | **198** | **182** | **165** | **149** |

The addition of free fatty acid increases the amount of readily oxidizable matter in the system and thereby places an additional stress on the antioxidant. Depending upon the quantities of free fatty acid one employs, it is preferred to use some stronger acid, such as citric, when a free fatty acid is employed.

Irganox 1010 is quite sensitive to soap pH and will maintain its structural integrity only in soaps essentially free of free alkali. A similar issue can present itself with compounds of group b. The excess alkali in soap formulations can be effectively neutralized by the in-situ "superfatting" with strong acids or with free fatty acids. Any quantity of acid which brings about at least essential neutralization of the free alkali can be employed. Quantities of free acid such as citric, acetic, and tartaric as low as about 0.1 wt.% can be employed with effectiveness. The amount of free fatty acid can also vary considerably although generally at least about 3 or 5 wt.% free fatty acid such as stearic, coco, myristic and the like can be employed, preferably at least about 7 wt.% of the composition. The final pH of the composition, as measured by 1 wt.% solid composition in water is generally from about 6.5 to about 10.3. Too acidic a pH should be avoided as well since compound cleavage can also occur.

## Claims

**1.** A cleansing composition which comprises a cleansing effective amount of a long chain alkyl or alkenyl containing surfactant, wherein by long chain is meant at least 8 carbon atoms, or mixtures thereof and a color stabilizing effective amount of a compound selected from the group consisting of

a.

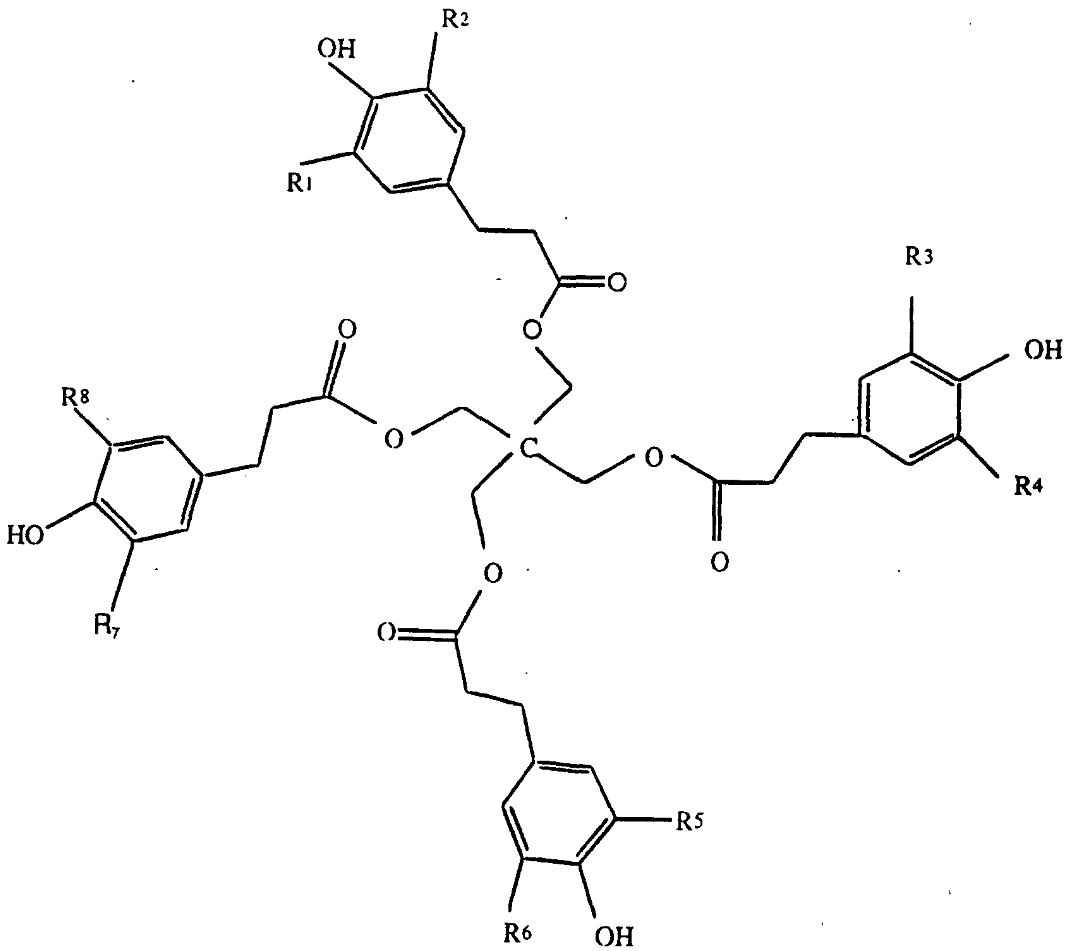

wherein $R_1$, $R_2$, $R_3$, $R_4$, $R_5$, $R_6$, $R_7$ and $R_8$ are the same or different and are isopropyl or tertiary butyl,

b.

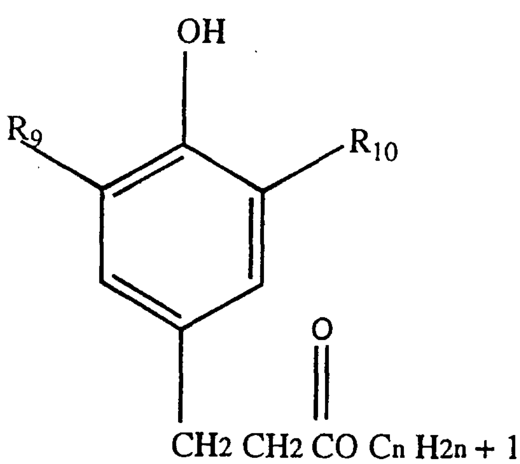

wherein $R_9$ and $R_{10}$ are the same or different and are isopropyl or tertiary butyl and n is 8 to 20, or

c.

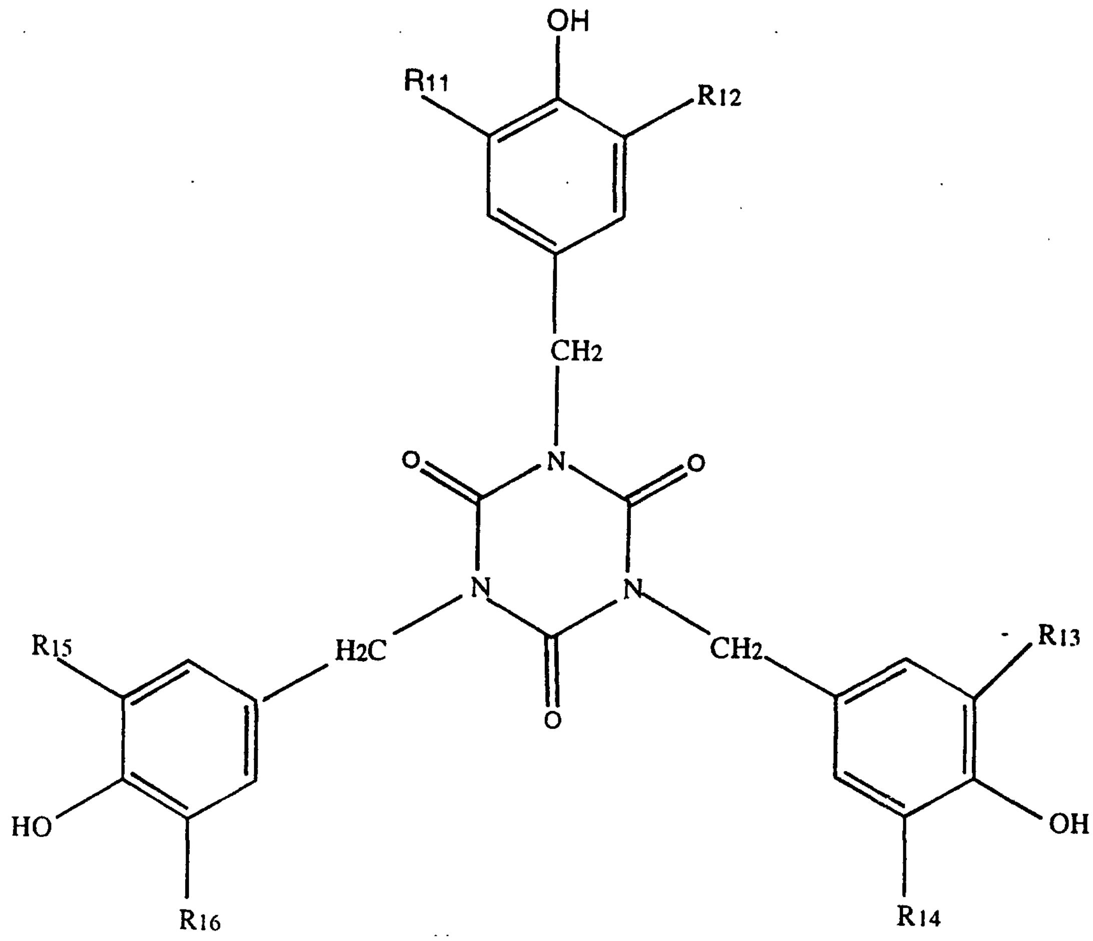

wherein $R_{11}$, $R_{12}$, $R_{13}$, $R_{14}$, $R_{15}$ and $R_{16}$ are the same or different and are isopropyl or tertiary butyl.

2. The composition in accordance with claim 1 wherein each R in each group a is the same, group b is the same, and group c is the same.

3. The composition in accordance with claim 2 wherein each R group is tertiary butyl.

4. The composition in accordance with claim 3 wherein the cleansing composition is a solid.

5. The composition in accordance with claim 4 wherein the solid can be hand held.

6. The composition in accordance with claim 5 wherein the solid is in a bar shape.

7. The composition in accordance with claim 3 wherein the compound is present in the composition in from 25 to 500 ppm of the composition.

8. The composition in accordance with claim 6 or with claim 7 wherein the compound is present in the composition in from 50 to 300 ppm.

9. The composition in accordance with claim 3 or with claim 6 wherein essentially all of the free alkali in the composition is neutralized.

10. The composition in accordance with claim 3 or with claim 6 wherein there is sufficient strong acid, free fatty acid or mixtures thereof to essentially overcome the hydrolytic effects of free alkali on the compound.

**11.** The composition in accordance with claim 6 wherein the pH of the bar, as measured in a 1% solution in water is 6.5 to 10.3.

**12.** The composition in accordance with claim 6 wherein the surfactant is soap.

**13.** The composition in accordance with claim 12 wherein the compound is present in from 25 to 500 ppm of the composition.

**14.** The composition in accordance with claim 13 wherein there is sufficient acid to essentially neutralize any free alkali present.

**15.** The composition in accordance with claim 14 wherein the pH of the solid, as measured in a 1% solution in water is from 6.5 to 10.3.

**16.** The composition in accordance with claim 3 wherein the cleansing composition is suitable for cleansing teeth.

**17.** The cleansing composition in accordance with claim 1 suitable for cleansing the oral cavity.

**18.** The cleansing composition in accordance with claim 17 suitable for cleansing teeth found in the oral cavity.

**19.** The composition in accordance with claim 9, with claim 10 or with claim 14 wherein the compound in the composition is selected from group a or b.

**20.** The composition in accordance with any one of claims 1-3, 5, 7, 9, 10-12 and 14, wherein the compound is selected from group a.

**Patentansprüche**

**1.** Reinigungszusammensetzung, die eine reinigend wirkende Menge eines langkettiges Alkyl oder Alkenyl enthaltenden Tensids, wobei mit langkettig mindestens 8 Kohlenstoffatome gemeint ist, oder von Mischungen davon und eine farbstabilisierend wirkende Menge einer Verbindung ausgewählt aus der Gruppe bestehend aus

a.

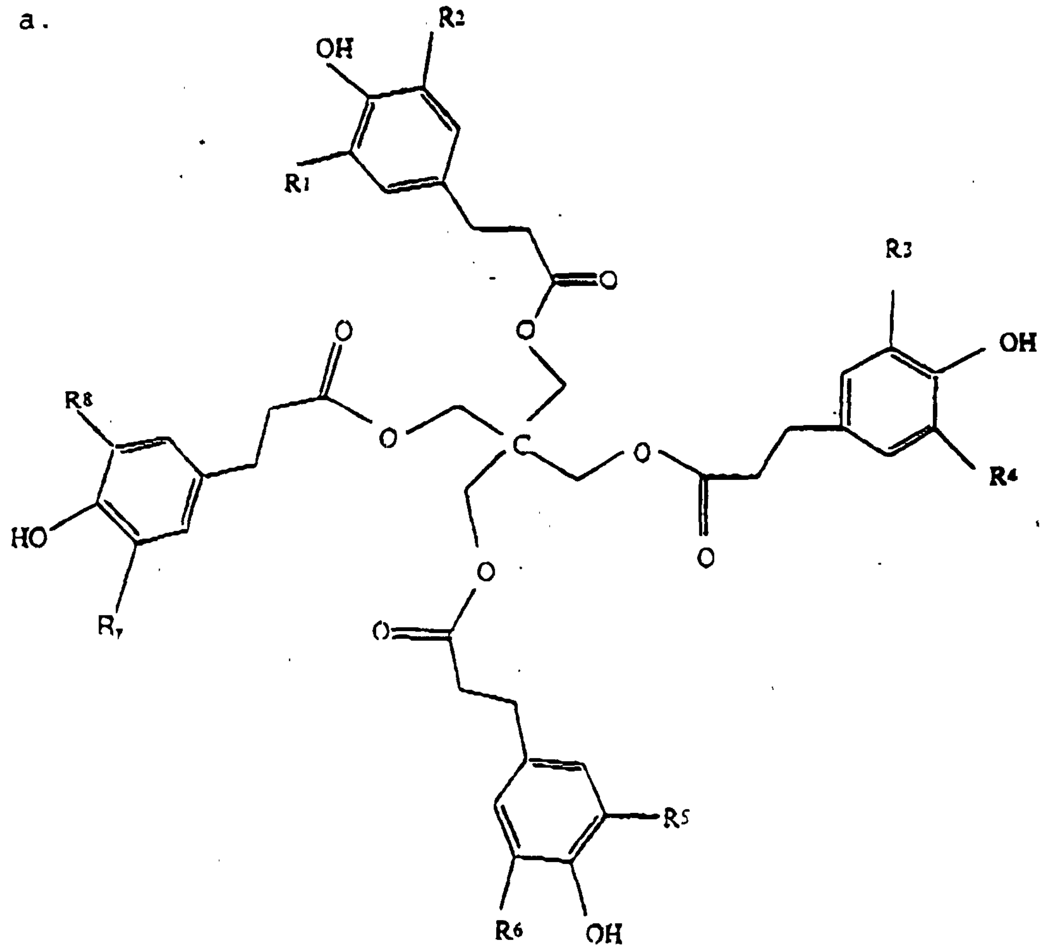

in der $R_1$, $R_2$, $R_3$, $R_4$, $R_5$, $R_6$, $R_7$ und $R_8$ gleich oder verschieden sind und Isopropyl oder tert.-Butyl sind,

b.

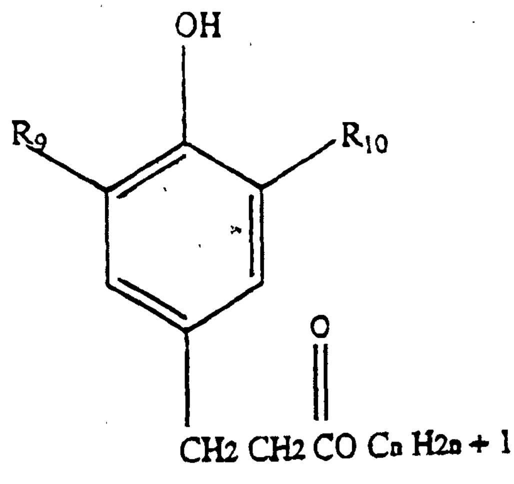

in der $R_9$ und $R_{10}$ gleich oder verschieden sind und Isopropyl oder tert.-Butyl sind und n 8 bis 20 ist, oder

c.

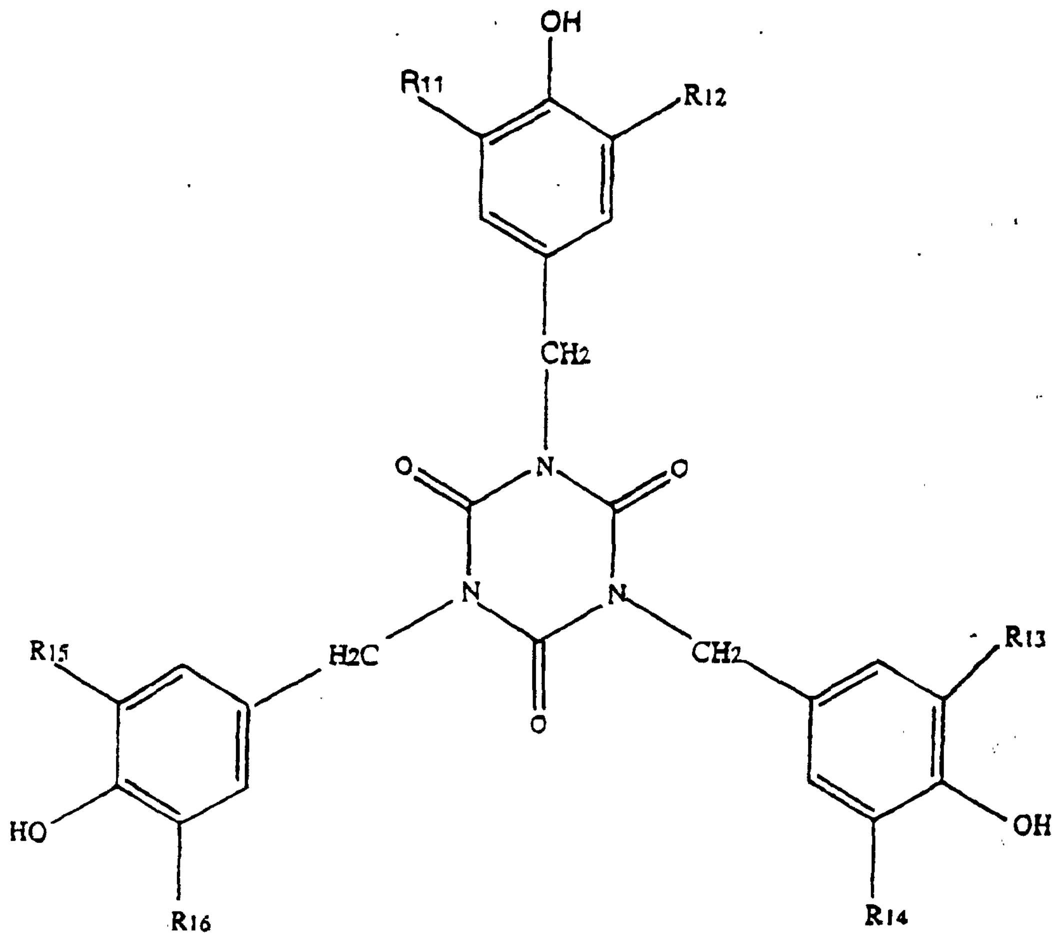

enthält, in $R_{11}$, $R_{12}$, $R_{13}$, $R_{14}$, $R_{15}$ und $R_{16}$ gleich oder verschieden sind und Isopropyl oder tert.-Butyl sind.

2. Zusammensetzung nach Anspruch 1, bei der jedes R in jeder Gruppe a das gleiche ist, in jeder Gruppe b das gleiche ist und in jeder Gruppe c das gleiche ist.

3. Zusammensetzung nach Anspruch 2, bei der jedes R tert.-Butyl ist.

4. Zusammensetzung nach Anspruch 3, bei der die Reinigungszusammensetzung ein Feststoff ist.

5. Zusammensetzung nach Anspruch 4, bei der der Feststoff in der Hand gehalten werden kann.

6. Zusammensetzung nach Anspruch 5, bei der der Feststoff in einer Riegelform vorliegt.

7. Zusammensetzung nach Anspruch 3, bei der die Verbindung in der Zusammensetzung in etwa 25 bis etwa 500 ppm der Zusammensetzung vorhanden ist.

8. Zusammensetzung nach Anspruch 6 oder Anspruch 7, bei der die Verbindung in der Zusammensetzung in etwa 50 bis 300 ppm vorhanden ist.

9. Zusammensetzung nach Anspruch 3 oder Anspruch 6, bei der im Wesentlichen alles freie Alkali in der Zusammensetzung neutralisiert ist.

10. Zusammensetzung nach Anspruch 3 oder Anspruch 6, bei der ausreichend starke Säure, freie Fettsäure oder

Mischungen derselben vorhanden ist/sind, um im Wesentlichen die hydrolytischen Effekte von freiem Alkali auf die Verbindung zu überwinden.

**11.** Zusammensetzung nach Anspruch 6, bei der der pH-Wert des Riegels, wie gemessen in einer 1%igen Lösung in Wasser, 6,5 bis 10,3 ist.

**12.** Zusammensetzung nach Anspruch 6, bei der das Tensid Seife ist.

**13.** Zusammensetzung nach Anspruch 12, bei der die Verbindung in 25 bis 500 ppm der Zusammensetzung vorhanden ist.

**14.** Zusammensetzung nach Anspruch 13, bei der ausreichend Säure vorhanden ist, um im Wesentlichen jegliches freie vorhandene Alkali zu neutralisieren.

**15.** Zusammensetzung nach Anspruch 14, bei der der pH-Wert des Feststoffs, wie gemessen in einer 1%igen Lösung in Wasser, 6,5 bis 10,3 ist.

**16.** Zusammensetzung nach Anspruch 3, bei der die Reinigungszusammensetzung für die Reinigung von Zähnen geeignet ist.

**17.** Reinigungszusammensetzung nach Anspruch 1, die für die Reinigung der Mundhöhle geeignet ist.

**18.** Reinigungszusammensetzung nach Anspruch 17, die für die Reinigung von Zähnen geeignet ist, die im Mundraum vorhanden sind.

**19.** Zusammensetzung nach Anspruch 9, Anspruch 10 oder Anspruch 14, bei der die Verbindung in der Zusammensetzung ausgewählt ist aus Gruppe a oder b.

**20.** Zusammensetzung nach einem der Ansprüche 1 bis 3, 5, 7, 9, 10 bis 12 und 14, bei der die Verbindung in der Zusammensetzung ausgewählt ist aus Gruppe a.

## Revendications

**1.** Composition de nettoyage qui comprend une quantité efficace de nettoyage d'un tensioactif contenant une longue chaîne alkyle ou alkényle, dans lequel, par longue chaîne, on entend au moins 8 atomes de carbone, ou un mélange de ces tensioactifs et une quantité efficace stabilisante de la couleur d'un composé choisi parmi le groupe consistant en :

a.

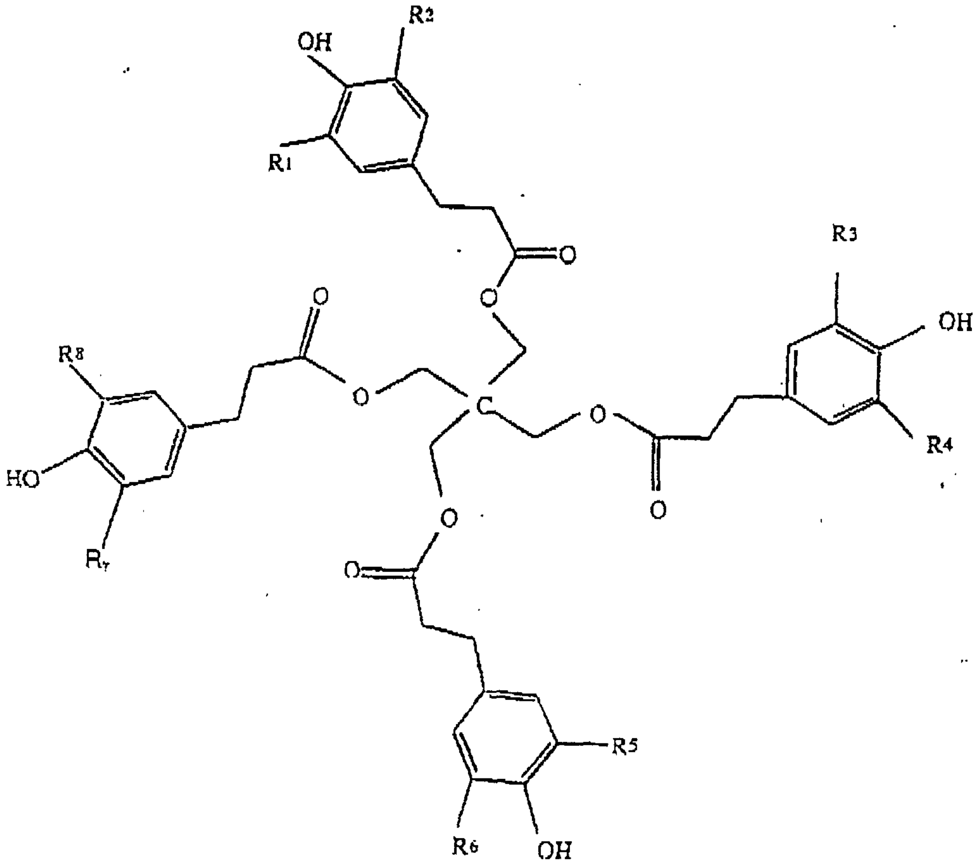

dans lequel $R_1$, $R_2$, $R_3$, $R_4$, $R_5$, $R_6$, $R_7$ et $R_8$ sont identiques ou différents et représentent le radical isopropyle ou butyle tertiaire,

b.

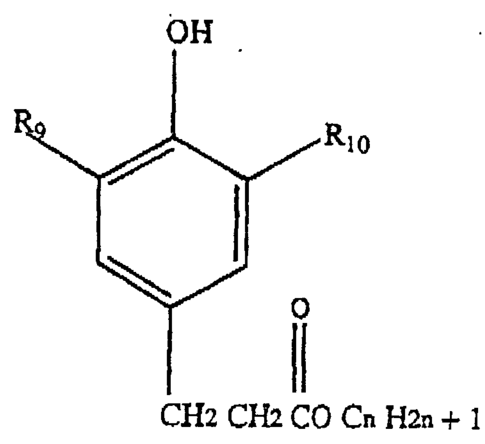

dans lequel $R_9$ et $R_{10}$ sont identiques ou différents et représentent le radical isopropyle ou butyle tertiaire et n vaut de 8 à 20, ou

c.

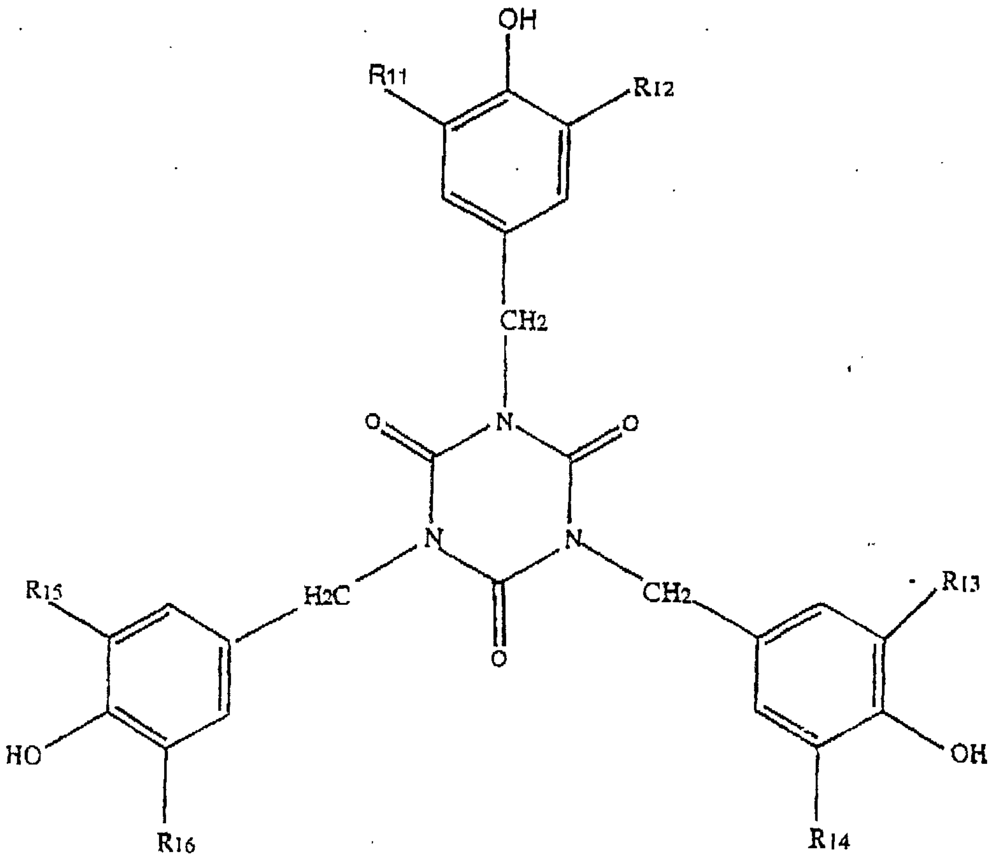

dans lequel $R_{11}$, $R_{12}$, $R_{13}$, $R_{14}$, $R_{15}$ et $R_{16}$ sont identiques ou différents et représentent le radical isopropyle ou butyle tertiaire.

**2.** Composition selon la revendication 1, dans laquelle chaque R dans chaque groupe a est identique, dans chaque groupe b est identique et dans chaque groupe c est identique.

**3.** Composition selon la revendication 2, dans laquelle chaque groupe R est le radical butyle tertiaire.

**4.** Composition selon la revendication 3, dans laquelle la composition de nettoyage est solide.

**5.** Composition selon la revendication 4, dans laquelle le solide est portable.

**6.** Composition selon la revendication 5, dans laquelle le solide est sous forme d'une barre.

**7.** Composition selon la revendication 3, dans laquelle le composé est présent dans la composition en une quantité de 25 à 500 ppm de la composition.

**8.** Composition selon la revendication 6 ou 7, dans laquelle le composé est présent en une quantité de 50 à 300 ppm dans la composition.

**9.** Composition selon la revendication 3 ou 6, dans laquelle essentiellement tous les alcalins libres dans la composition sont neutralisés.

**10.** Composition selon la revendication 3 ou 6, dans laquelle il y a suffisamment d'acide fort, d'acide gras libre ou leur mélange pour surmonter essentiellement les effets hydrolytiques des alcalins libres sur le composé.

**11.** Composition selon la revendication 6, dans laquelle le pH de la barre mesuré dans une solution à 1 % dans l'eau est de 6,5 à 10,3.

**12.** Composition selon la revendication 6, dans laquelle le tensioactif est un savon.

**13.** Composition selon la revendication 12, dans laquelle le composé est présent en une quantité de 25 à 500 ppm de la composition.

**14.** Composition selon la revendication 13, dans laquelle est présent suffisamment d'acide pour neutraliser essentiellement les alcalins libres présents.

**15.** Composition selon la revendication 14 dans laquelle le pH du solide mesurée en solution à 1% dans l'eau est de 6,5 à 10,3.

**16.** Composition selon la revendication 3 dans laquelle la composition de nettoyage est adaptée pour nettoyer les dents.

**17.** Composition de nettoyage selon la revendication 1 adaptée pour nettoyer la cavité orale.

**18.** Composition de nettoyage selon la revendication 17 adaptée pour nettoyer les dents si situant dans la cavité orale.

**19.** Composition selon la revendication 9, 10 ou 14, dans laquelle le composé de la composition est choisi dans le groupe constitué par a et b.

**20.** Composition selon l'une quelconque des revendications 1 à 3, 5, 7, 9, 10 à 12 et 14, dans laquelle le composé est choisi parmi le groupe a.